Europäisches Patentamt

European Patent Office

Office européen des brevets

(19)

(11) **EP 1 054 660 B1**

(12) **FASCICULE DE BREVET EUROPEEN**

(45) Date de publication et mention
de la délivrance du brevet:
**05.12.2001 Bulletin 2001/49**

(21) Numéro de dépôt: **99901695.9**

(22) Date de dépôt: **03.02.1999**

(51) Int Cl.$^7$: **A61K 7/48**

(86) Numéro de dépôt international:
**PCT/FR99/00220**

(87) Numéro de publication internationale:
**WO 99/40896 (19.08.1999 Gazette 1999/33)**

(54) **COMPOSITION COSMETIQUE CONTENANT UN COMPOSE A ACTIVITE STIMULATRICE DE LA PRODUCTION D'INTERLEUKINE-6**

KOSMETISCHE ZUSAMMENSETZUNG, ENTHALTEND EINE MISCHUNG ZUR STIMULATION DER INTERLEUKIN-6 PRODUKTION

COSMETIC COMPOSITION CONTAINING A COMPOUND WITH ACTIVITY STIMULATING INTERLEUKIN-6

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **11.02.1998 FR 9801637**

(43) Date de publication de la demande:
**29.11.2000 Bulletin 2000/48**

(73) Titulaire: **SANOFI-SYNTHELABO**
**75013 Paris (FR)**

(72) Inventeurs:
• **CASELLAS, Pierre**
**F-34090 Montpellier (FR)**
• **DEROCQ, Jean-Marie**
**F-34570 Murviel les Montpellier (FR)**
• **PEREILLO, Jean-Marie**
**F-31120 Portet sur Garonne (FR)**

(74) Mandataire: **Thouret-Lemaitre, Elisabeth**
**Sanofi-Synthélabo**
**Service Brevets**
**174, avenue de France**
**75013 Paris (FR)**

(56) Documents cités:
**EP-A- 0 335 554**

• **DATABASE WPI Week 9510 Derwent Publications Ltd., London, GB; AN 95-070208 XP002088375 "Cosmetic material-constains collagenase inhibitor and controls reduction of dermal collagen and loss of skin elasticity" A & JP 06 345636 A (NONOGAWA SHOJI YG) 20 décembre 1994**
• **PATENT ABSTRACTS OF JAPAN vol. 016, no. 194 (C-0938), 11 mai 1992 & JP 04 029916 A (SHISEIDO CO LTD), 31 janvier 1992**
• **MOLLER A ET AL: "Regulation of monocyte and keratinocyte interleukin 6 production by transforming growth factor beta." EXPERIMENTAL DERMATOLOGY, (1994 DEC) 3 (6) 314-20. JOURNAL CODE: B06. ISSN: 0906-6705., XP002088374 Denmark**
• **PATENT ABSTRACTS OF JAPAN vol. 013, no. 072 (C-570), 17 février 1989 & JP 63 264513 A (LION CORP), 1 novembre 1988**

**Description**

La présente invention concerne une composition cosmétique contenant un composé actif capable de stimuler *in vivo* la production d'interleukine-6 (IL-6) par les kératinocytes humains.

Parmi le large spectre de cytokines produites par les cellules, la stimulation spécifique de la synthèse d'IL-6 représente un intérêt particulier pour l'application en cosmétologie. Outre ses effets activateurs sur l'immunité cellulaire et humorale, l'IL-6 prend une part active dans les mécanismes de régénération de l'épiderme. *In vitro*, l'IL-6 stimule la synthèse de collagène ainsi que la prolifération des kératinocytes et des fibroblastes. *In vivo,* chez des souris transgéniques sur-exprimant l'IL-6 au niveau cutané, on observe un épaississement du stratum corneum. Cette augmentation de la couche superficielle de l'épiderme survient sans manifestation pro-inflammatoire et rendrait compte de l'amélioration de la protection de la peau contre les lésions locales. L'ensemble de ces données, issu de la littérature, indique que l'IL-6 favorise la réparation des dommages causés à l'épiderme et ralentit le vieillissement cutané.

On pourra ainsi se référer à K. Yoshisaki *et al.*, Cytokine, 1990, *2*, 381-387 ; R.M. Grossman *et al.*, Proc. Natl. Acad. Sci. USA, 1989, *86*, 6367-6371 ; Kirbauer *et al.*, J. Immunol., 1989, *142*, 1922-1928 ; K. Turken *et al.*, Proc. Natl. Acad. Sci. USA, 1992, 89, 5068-5072 ; J. Taylor-Papadimetriou *et al.*, Cell. Differ., 1982, *11*, 169-180 ; L. Aarden *et al.*, Lymphokines, 1985, *10*, 175-182. Il est bien connu que lors du vieillissement cutané, d'une part au niveau de l'épiderme, la couche cornée est épaissie mais l'épidermopoïèse est diminuée et la cohésion cellulaire au niveau de la membrane basale est altérée. Il est donc important d'apporter à la peau pour l'aider à lutter contre le vieillissement cutané un actif qui soit capable de stimuler la prolifération des kératinocytes de façon à amener un épaississement de l'épiderme. D'autre part, au niveau du derme, les cellules responsables de la construction de l'architecture dermique, les fibroblastes ont une activité de plus en plus réduite avec comme conséquence une diminution de la synthèse de collagène, d'élastine et de glycosominoglycane. Parallèlement, les effets de l'exposition à la lumière vont entraîner une dégradation accélérée des fibres dermiques existantes (vieillissement actinique). L'ensemble de ces effets conduit à une structure dermique désorganisée, qui enlève à la peau ses qualités mécaniques d'élasticité, de fermeté et de tonicité et favorise ainsi l'apparition des rides.

Il est donc important pour s'opposer aux effets du vieillissement, d'apporter à la peau un actif qui va stimuler les fibroblastes et les aider à reconstruire leur environnement. Cet effet pour être utile ne doit pas s'accompagner en parallèle d'une augmentation de l'activité enzymatique de dégradation des fibres, en particulier de la collagénase. Il ne doit pas non plus modifier les fonctions naturelles de la peau.

Le document JP-A-06 345636 décrit une composition cosmétique destinée à contrôler la réduction de collagène dans le derme et comprenant un inhibiteur de collagénase et une substance favorisant la synthèse de collagène.

Le document JP-A-04 029916 décrit une composition pour la peau comprenant un facteur de croissance cellulaire, notamment un facteur TGF-$\beta$, de la saikosaponine $b_1$ et/ou de la saikosaponine $b_2$.

Le document EP-A-0 335 554 décrit une composition cosmétique ou pharmaceutique comprenant un facteur de repousse des cheveux choisi parmi les facteurs TGF-$\alpha$, TGF-$\beta$ ou IGF 1.

Le document Moller et al, Experimental Dermatology, (1994 Dec), 3 (6), 314-320 met en évidence la stimulation de la production d'IL-6 par le facteur TGF-$\beta$ dans les cellules sanguines mononucléaires et les kératinocytes.

Le document JP-A-63 264513 décrit une composition cosmétique adaptée notamment pour la peau et les cheveux, contenant le produit de réaction obtenu en traitant une n-paraffine avec une levure capable de produire des graisses ou des huiles.

Il a maintenant été trouvé que les composants ou extraits capables de stimuler *in vivo* la production d'IL-6 par les kératinocytes humains constituent des "actifs" qui peuvent être utilisés pour la préparation de compositions cosmétiques ; plus particulièrement, il a été trouvé que des compositions cosmétiques contenant un composé capable de stimuler *in vivo* la production d'IL-6 par les kératinocytes humains peuvent être utilisés dans la restructuration du tissu cutané sans pour autant interférer avec les fonctions naturelles de celui-ci. Les composés à activité stimulatrice de l'IL-6 par les kératinocytes humains aussi appelé ci-après "actifs" sont montrés capables d'induire une action propre sur la stimulation de la prolifération des kératinocytes (action autocrine de l'IL-6 sur les kératinocytes décrite dans la littérature).

Par ailleurs, les composés à activité stimulatrice de l'IL-6 par les kératinocytes humains se sont montrés capables d'induire par leur action sur le fibroblaste une augmentation de la tonicité de l'environnement dermique de cette cellule. Ces composés induisent même une légère augmentation du nombre de fibres de collagène, sans augmentation de l'activité collagénasique.

Ainsi, un premier objet de la présente invention concerne une composition cosmétique contenant un composé capable de stimuler *in vivo* la production d'interleukine-6 par les kératinocytes humains en mélange avec un excipient cosmétique.

Le composé à activité stimulatrice de la production d'IL-6, ou actif, contenu dans la composition cosmétique peut être un composé non peptidique, un peptide, un extrait de cellules ou de tissus d'origine végétale ou un produit obtenu par fermentation d'un micro-organisme, par exemple d'une bactérie ou d'un champignon et plus particulièrement d'une levure.

On décrira ci-après les caractéristiques de la souche SEBR 2002, ainsi que le procédé d'obtention de l'extrait à activité stimulatrice de la production d'interleukine-6 par les kératinocytes.

**Souche**

*Découverte*

L'organisme producteur des extraits à activité stimulatrice de la production d'IL-6 par les kératinocytes humains selon cet aspect particulier de la présente invention, est une souche de levure qui a été isolée à partir d'un échantillon d'eau de gisement prélevé sur un forage pétrolier dans le Loiret (France), auquel on a attribué le numéro interne SEBR 2002. Un échantillon de ce micro-organisme a été déposé le 11 février 1997 auprès de la C.N.C.M. de l'Institut Pasteur où il a été enregistré sous la référence I 1844.

Les caractéristiques biochimiques de ce micro-organisme ont été déterminées sur galeries API 50 CH (spécifiques des sucres), commercialisés par BioMérieux et par le test biologique YT (spécifique des levures commercialisées par Biolog Inc. USA). On a ainsi déterminé que ce micro-organisme appartient à la famille des *Basidiomycetes,* genre *Rhodotorula.*

Il s'agit d'une levure polymorphe, mésophile. Elle se développe bien à 28°C sur un milieu de culture YPG (agar yeast peptone glucose), la coloration des colonies est rose-orangé.

Cet organisme, présente des caractères qui permettent de l'apparenter à l'espèce minuta.

Cette souche de levure *Rhodotorula sp*, déposée auprès de la C.N.C.M. de l'Institut Pasteur sous le numéro I 1844 et ses mutants producteurs constituent également un objet de la présente invention.

L'isolement de cette nouvelle souche a été effectué en suivant la méthode usuelle qui consiste à diluer une petite quantité d'eau du prélèvement à différentes concentrations et à étaler un petit volume de chaque dilution sur la surface d'une boîte de Petri contenant un milieu nutritif gélosé. Après une incubation de quelques jours à 28°C, qui permet aux micro-organismes de se développer, les différentes colonies sont séparément prélevées et repiquées sur les géloses nutritives afin d'en obtenir des cultures plus abondantes.

Après cultures sur milieu nutritif gélosé et plusieurs repiquages successifs qui permettent d'obtenir une culture abondante et pure de la souche d'intérêt, on fabrique un lot 0 de conservation de la souche mère puis des lots de semence primaire et secondaire.

Pour cela, on prépare une suspension des levures à partir d'une culture sur milieu nutritif gélosé en boîte de Pétri et d'un milieu de reprise ; ce milieu contient un cryoprotectant permettant d'assurer une bonne viabilité du microorganisme lors de la conservation par congélation.

La suspension de levures obtenues est répartie dans des cryotubes qui sont conservés à -80°C : ces tubes constituent le lot 0.

En suivant le même protocole, mais à partir d'un tube du lot 0, on prépare un lot de semence primaire.

Puis toujours selon le même protocole on prépare un lot de semence secondaire à partir d'un cryotube du lot de semence primaire.

La fabrication des lots de semences 0,1 et 2 assure une accessibilité durable de la souche et donc de l'activité recherchée.

**Procédé**

Le procédé de préparation des extraits à activité stimulatrice de la production d'IL-6 par les kératinocytes humains consiste à cultiver cette nouvelle souche I 1844, ou ses mutants producteurs, sur un milieu et dans des conditions de culture appropriée et à extraire ensuite la fraction active.

La fraction active peut se trouver dans la biomasse ou dans le surnageant.

**Fermentation**

La culture de la souche I 1844 peut être effectuée par toute méthode de culture aérobie. On utilise à cette fin, les différents types d'appareils qui sont d'un usage courant dans l'industrie des fermentations. On peut, en particulier, adopter la démarche suivante pour la conduite des opérations.

**Culture en fiole**

A partir du lot de semences secondaire, on ensemence des boîtes de Pétri qui, après deux à trois jours d'incubation, fournissent une suspension de levures qui est utilisée pour ensemencer des fioles Erlenmeyer agitées contenant un milieu approprié. On peut aussi ensemencer directement la fiole agitée avec un tube du lot de semence. La culture en fioles agitées peut durer de un à trois jours.

La production d'activité est observée par extraction de la biomasse obtenue par filtration ou centrifugation avec des solvants organiques.

Dès le premier étage de culture en fioles, il peut être avantageux de réaliser deux étages de culture successifs : un premier étage pour multiplier les cellules et propager la biomasse, un second pour la production. Dans ce dernier cas une durée d'un à deux jours est suffisante pour le premier étage.

L'activité de stimulation de la production d'IL-6 par les kératinocytes humains, contenue dans les extraits de biomasse des cultures est exprimée par un facteur ou index de stimulation nommé $IS_{IL-6}$ (IS) calculé en faisant le rapport des quantités d'IL-6 produite par induction par les extraits à la valeur mesurée au niveau basal (non induit) :

$$IS_{IL-6} = \frac{Production\ IL\text{-}6\ induite}{Production\ IL\text{-}6\ basale}$$

Un extrait de biomàsse est considéré comme actif lorsque testé à 1‰ en concentration finale par rapport à l'extrait sec, sur kératinocytes SVK 14, il produit un IS compris entre les valeurs 1,2 et 6 de préférence de 1,5 à 4,5.

Un litre de culture permet d'obtenir environ de 10 à 50 ml d'extrait ayant un indice de stimulation compris entre 1,5 et 4,5, pour une dilution au 100ème (1/100).

L'activité recherchée peut être obtenue par extraction de la biomasse des cultures en fioles mais il paraît avantageux, afin d'obtenir l'activité recherchée en plus grande quantité, de réaliser une culture en fermenteur et d'extraire ensuite la biomasse de celle-ci.

### Culture en fermenteur

Le fermenteur est ensemencé par une culture en fiole agitée âgée de 1 à 2 jours, il est préférable que la culture soit encore en phase exponentielle.

En fermenteur, suivant les conditions de culture utilisées, l'activité peut être observée dès les premières heures de la culture mais il est avantageux d'attendre que la phase de croissance stationnaire soit atteinte avant de procéder à l'extraction.La culture de I 1844 en fermenteur permet de mieux contrôler les conditions de culture qui sont décrites ci-après par exemple le pH et l'aération.

L'activité stimulatrice par les kératinocytes obtenues en fermenteur peut varier, selon les conditions de culture appliquées.Un litre de culture permet d'obtenir après extraction de la biomasse environ 50 ml d'extrait présentant un IS compris entre 1,5 et 4,5 pour une dilution au 100ème (1/100).

### Conditions de culture

Le milieu de culture utilisé dans le procédé de fermentation doit contenir au moins une source de carbone assimilable, une source d'azote assimilable et des éléments minéraux. Comme sources de carbone assimilable, on peut utiliser par exemple, des hydrates de carbone, tels que le glucose, le mannose, le maltose, des dextrines, le glycérol, les acides aminés et les protéines.

Comme sources de carbone assimilable, on peut aussi utiliser les acides acétique, subérique, citrique, propionique, succinique et 2-cétoglutarique ou des huiles animales ou végétales.

Les meilleures sources d'azote assimilable se trouvent parmi les protéines, les peptones et les acides aminés. Ces sources comprennent, par exemple, la caséïne, la lactalbumine, le gluten et leurs hydrolysats, la farine de poisson, les extraits de levure ou les peptones.

La production de biomasse peut être augmentée par l'adjonction, au cours de la culture, de l'un et/ou de l'autre de ces deux principaux substrats.

Parmi les éléments minéraux ajoutés au milieu de culture pour assurer la croissance du micro-organisme et optimiser l'assimilation des sources de carbone et d'azote par les cellules du micro-organisme, on peut citer des sels de potassium, de sodium, de fer, de magnésium, de calcium, ou de manganèse ainsi que les composés du phosphore tels que les phosphates et les oligo-éléments.

La culture est agitée et aérée pendant une durée variant entre 10 heures et 60 heures ce qui permet d'obtenir des résultats avantageux.

De préférence, on maintient le pH du milieu de culture à une valeur légèrement acide et la température optimum d'incubation se situe entre 23°C et 38°C, l'intervalle préféré étant 25°C à 33°C.

Les conditions de culture telles que la composition et le pH du milieu, la température d'incubation, la vitesse d'agitation ainsi que l'aération de la fermentation peuvent varier dans de larges limites et sont choisies de façon à obtenir les meilleurs résultats possibles.

*Extraction*

L'obtention de l'extrait actif présentant une activité stimulatrice de la production d'IL-6 par les kératinocytes demande plusieurs étapes d'extraction.

Une première étape consiste à séparer la biomasse du reste du moût, pour cela on peut utiliser la centrifugation, la microfiltration tangentielle, la filtration à tambour rotatif ou toute autre méthode habituellement utilisée par l'homme du métier pour séparer la biomasse d'un moût de fermentation. La biomasse est ensuite mise en contact pendant quelques heures avec un mélange de solvants permettant d'extraire les molécules à caractère hydrophobe.

La durée de mise en contact préférée est une nuit, soit 15 heures environ.

La biomasse et la phase organique chargée sont ensuite séparées par filtration frontale, la biomasse est éliminée.

La phase organique est ensuite évaporée sous vide à une température variant de la température ambiante à 50°C jusqu'à l'obtention d'un extrait sec.

*Préparation de l'extrait actif*

L'extrait sec ainsi obtenu peut être repris dans différents solvants habituellement utilisés en cosmétologie.

La concentration de reprise est choisie pour permettre une parfaite dissolution de l'extrait et pour être compatible avec l'utilisation ultérieure.

L'extrait stimulateur de la production d'IL-6 par les kératinocytes, à raison de 5 à 10 % d'extrait sec selon l'activité, est introduit dans un mélange éthanol/eau 50/50 (v/v) qui constitue le solvant préféré selon l'invention. Si l'on désire obtenir une poudre au lieu d'une solution, on peut simplement lyophiliser l'extrait sec.

Un dosage d'activité stimulatrice de la production d'IL-6 par les kératinocytes réalisé sur une partie aliquote de chaque extrait obtenu permet d'évaluer son activité et de vérifier la reproductibilité du procédé.

Afin d'éliminer toute trace de biomasse résiduelle et d'assurer sa stabilité microbiologique, l'extrait est filtré sur une membrane ayant un seuil d'exclusion de 0,2 μm et réparti aseptiquement dans des flacons stériles. La préparation aseptique de ces solutions permet d'éviter l'ajout de conservateur.Différents essais biochimiques ont été réalisés et ont permis de mettre en évidence que les extraits issus de ce nouveau micro-organisme I 1844 ont une activité stimulatrice de la production d'IL-6 par les kératinocytes, très intéressante. Leur puissante activité a été démontrée sur différents essais prédictifs d'une réparation des différents dommages causés à l'épiderme et du ralentissement du vieillissement cutané.

La caractérisation de l'effet stimulant de l'extrait I 1844 sur la synthèse *in vitro* de l'IL-6 par les kératinocytes humains a été réalisée comme suit.

**Extrait I 1844 et produits utilisés**

Différents échantillons de l'extrait I 1844, issus de différentes cultures en fermenteur réalisés dans des conditions similaires ont été utilisés. Ces échantillons ont été conservés à 4°C dans un mélange éthanol/eau, 50/50 (v/v) à la concentration de 0,1 g/g d'extrait sec, puis dilués à la concentration finale souhaitée au moment de l'utilisation.

Le produit de référence pour l'induction de la synthèse de l'IL-6 a été le LPS (*E. Coli* 055:B5) Sigma, conservé à -20°C à la concentration de 10 mg/ml dans du PBS. La polymyxineB, inhibiteur de LPS, a été fournie par Sigma.

**Dilution des produits**

Les produits ont été dilués dans le milieu d'incubation des kératinocytes (cf. ci-dessous). Sauf indication contraire les extraits I 1844 préparés comme indiqué ci-dessus ont été évalués à la concentration finale de 1 ‰ par rapport à l'extrait sec. Le LPS a été testé a la concentration de 10 μg/ml.

**Modèle d'évaluation**

*Cellules*

La lignée de kératinocytes humains utilisée a été la lignée SVK 14 (J. Taylor-Papadimetriou *et al.*, Cell. Differ., 1982, *11*, 169-180), maintenue en culture dans le milieu DMEM contenant 4,5 g/l de glucose, 100 mmole de pyruvate de sodium, 50 U/ml de pénicilline, 50 μg/ml de streptomicyne et 10 % de sérum de veau foetal (SVF). A titre de comparaison, des lymphocytes normaux humains (PBMNC) purifiés sur gradient de Ficoll® et provenant de volontaires sains, ainsi que des kératinocytes provenant de la lignée A 431 (ATCC CRL 1555) et des kératinocytes normaux humains issus de chirurgie de plastie mammaire (Biopredic, Rennes) ont été cultivés dans le même milieu que celui décrit ci-dessus mais contenant en plus 10 mg/ml d'EGF et 50 µg d'extrait d'hypophyse bovine.

### Incubation avec les produits

Pour l'induction de l'IL-6, les cellules sont ensemencées en triplets sous un volume final de 200 µl à une densité de $5x10^4$ cellules/puits dans des microplaques de culture de 96 puits. Après 24 heures d'incubation à 37°C dans une atmosphère à 95 % d'air et de 5 % de $CO_2$, le milieu est éliminé et renouvelé par un même volume de milieu à 1 % de SVF contenant les produits aux concentrations indiquées. Au bout de 18 à 24 heures supplémentaires de culture, le surnageant de culture de chaque triplet est prélevé, rassemblé en pool et congelé à -20°C jusqu'à la détermination du contenu en cytokines.

### Dosages de cytokines

L'IL-6, l'IL-1β, le TNFα et l'IL-8 ont été quantifiés à l'aide de kits de dosage ELISA (R&D, Abington, UK). Chaque surnageant a été évalué en doublets selon les indications du fabricant. La limite de détection de ces dosages est de 3 pg/ml. En plus du dosage immunologique de la protéine par la technique ELISA, la forme active de l'IL-6 a également été évaluée par dosage biologique sur cellules d'hybridome murin B9 (L. Aarden *et al.*, Lymphokines, 1985, 10, 175-182).

### Expression des résultats

Les résultats sont exprimés soit en valeur absolue de quantité de cytokines sécrétées (en pg/ml de protéine pour les dosages ELISA ou U/ml pour le dosage biologique B9 de l'IL-6 : une unité étant définie comme étant la quantité de cytokine induisant 50 % de l'effet prolifératif maximum des cellules B9), soit en index de stimulation IS tel que défini précédemment calculé comme étant le rapport entre la production de cytokine induite et la production basale :

$$IS_{IL-6} = \frac{\text{Production IL-6 induite}}{\text{Production IL-6 basale}}$$

### Résultats

L'extrait à activité stimulatrice de production d'IL-6 sera ci-après dénommé simplement "extrait I 1844". Le numéro de l'extrait utilisé pour chaque expérience ainsi que sa concentration sont indiqués.

### Effet de l'extrait I 1844 sur la stimulation de l'IL-6 kératinocytaire

L'extrait I 1844 stimule la synthèse d'IL-6 des kératinocytes SVK 14 de façon dépendante de la concentration. Considérant, comme seuil de positivité la quantité moyenne d'IL-6 basale produite + 3 SD (11,8 + [3x0,8] = 13,6pg/ml), l'extrait I 1844 stimule de façon significative la synthèse d'IL-6 à partir de concentration voisines de 0,12 % (IS = 1,23), l'$EC_{50}$ est égale à 0,25 % et l'effet maximum est obtenu à la concentration de 2 % pour laquelle la synthèse d'IL-6 est stimulée d'un facteur 2,3.
L'étude a été étendue à une autre lignée de kératinocytes humains (lignée A 431) ainsi qu'à des kératinocytes humains normaux. A l'instar de ce qui a été observé sur la lignée SVK 14, l'extrait I 1844 est capable de stimuler la production d'IL-6 d'un facteur supérieur à 2 aussi bien sur une autre lignée de kératinocytes (A 431) que sur une primo-culture de kératinocytes normaux.
L'effet de l'extrait I 1844 n'est donc pas limité aux lignées mais est généralisable aux cellules normales.

### Spécificité de l'effet de l'extrait I 1844

La spécificité de l'effet de l'extrait I 1844 a été vérifiée à différents niveaux en démontrant :

- que l'effet n'était pas dû à une contamination par du LPS bactérien dont la capacité à stimuler la production d'IL-6 est connue et que des cellules d'origine lymphoïde également capables de produire l'IL-6, étaient insensibles à l'extrait I 1844.
- que parmi les cytokines pouvant être synthétisées par les kératinocytes, seule la production d'IL-6 était stimulée sous l'influence de l'extrait I 1844.

La présence de polymyxine, inhibiteur de LPS, ne modifie pas l'effet inducteur de l'extrait I 1844 sur la production d'IL-6 des kératinocytes SVK 14, alors que la stimulation induite par le LPS est logiquement inhibée par ce composé. Ce résultat indique que l'effet observé avec l'extrait I 1844 n'est pas provoqué par une contamination due à une endotoxine bactérienne. De plus, l'évaluation réalisée par bio-essai B9 indique que la production d'IL-6 stimulée par l'extrait I 1844 est biologiquement active.

L'étude comparative sur des cibles cellulaires différentes montre que les lymphocytes sanguins (PBMNC), contrairement aux kératinocytes sont insensibles à l'effet à activité stimulatrice de l'extrait I 1844 sur la production d'IL-6. En revanche le LPS, capable de stimuler les deux types cellulaires est efficace dans les deux cas. Cette étude montre donc que l'extrait I 1844 stimule les kératinocytes sans affecter les cellules d'origine lymphoïde.

Enfin, l'extrait I 1844 stimule la synthèse d'IL-6 sans augmenter de façon concomitante le taux d'IL-1, d'IL-8 et de TNF$\alpha$. Parmi les cytokines, l'IL-8 revêt un caractère particulier puisqu'elle est capable par ses propriétés chimiotactiques de recruter au niveau local les polynucléaires neutrophiles. Elle joue donc un rôle important dans l'inflammation. L'extrait I 1844 stimule la production d'IL-6 sans augmenter la production des autres cytokines étudiées.

L'ensemble de ces données : absence d'effet LPS, ciblage des seuls kératinocytes et stimulation limitée à l'IL-6 permet de conclure à la spécificité d'action de l'extrait I 1844 et à l'absence d'effet pro-inflammatoire.

Ainsi, l'extrait I 1844 stimule reproductiblement la production d'IL-6 kératinocytaire. Cet effet est observé aussi bien sur des kératinocytes humains établis en lignées que sur des kératinocytes normaux humains issus de primo-culture. La propriété de l'extrait I 1844 à stimuler la synthèse d'IL-6 par les kératinocytes est spécifique. Elle est due à une caractéristique intrinsèque à l'extrait I 1844 et non pas à une contamination exogène de type endotoxine bactérienne, elle n'affecte pas les cellules d'origine lymphoïde et ne s'observe parmi les cytokines testées que pour l'IL-6 : la spécificité de l'extrait I 1844 est double à la fois cellulaire et moléculaire. La caractérisation de l'effet à activité stimulatrice de l'extrait I 1844 sur l'IL-6 kératinocytaire a permis de définir comme critère d'activité pour tout échantillon évalué sur les cellules SVK14 des index de stimulation de l'IL-6 compris entre 1,5 et 4,5.

La caractérisation de l'effet de l'extrait I 1844 sur la contraction des fibres de collagène a été réalisée comme suit.

Le but de cette étude est de tester l'effet de l'extrait I 1844 sur la contraction des fibres de collagène. On utilise un gel de collagène réalisé par le mélange de fibroblastes et de collagène. Sous l'effet de tensions exercées par les cellules, le collagène s'organise en fibres. Ce phénomène se quantifie par la contraction du gel.

La lignée cellulaire utilisée est la souche MRC5, fibroblastes issus de poumon embryonnaire humain. Ils sont cultivés en milieu BME (Basal Eagle Medium) avec Glutamax I contenant 10 % de SVF, 1% de penicilinesteptomycine, 1% de fungizone et 1% de NEAA (acides aminés non essentiels) Gibco®.

Le collagène utilisé est du collagène de type I issu de tendons de queue de rat, en solution à 2 mg/ml dans l'acide acétique.

**Méthode d'obtention des gels**

*Préparation de la suspension cellulaire*

Après trypsinisation d'une culture de MRC5 obtenue sur une boîte de Pétri de 75 cm$^2$, la suspension cellulaire est préparée dans du DMEM contenant 10% de SVF et les antibiotiques à raison de $1.10^6$ cellules/ml de façon à obtenir 500 000 cellules par gel.

La préparation du mélange se fait *sur glace* afin de retarder la gélification.

Les différents constituants sont ajoutés sous agitation dans l'ordre et les proportions suivants :

| | |
|---|---|
| DMEM 1,76 X | 2,3 ml |
| Collagène type I | 1,5 ml |
| NaOH 0,1 N | 0,25 ml |
| SVF | 0,45 ml |
| Suspension cellulaire | 0,5 ml |

Ce mélange bien homogénéisé est réparti dans une boîte de Pétri de 5 cm de diamètre (NUNC type bactériologie) et incubé à 37°C. Le gel se forme dans les 10 minutes qui suivent. Le gel est décollé de la paroi de la boîte environ 2 heures après, à l'aide d'une lame de scalpel. Le diamètre des gels est alors mesuré à 24, 48 et 72 heures après formation à l'aide d'une règle graduée en millimètres.

**Traitement**

L'extrait I 1844 est dilué dans le milieu DMEM 1,76X lors de l'obtention du gel de façon à obtenir les concentrations finales de 0,1%, 0,2% et 0,5%. Une solution de phénol a la concentration finale de 6,4 g/l est utilisée comme témoin d'inhibition de la contraction.

**Analyse quantitative**

L'extrait I 1844 induit dès la concentration de 0,1% une augmentation significative de la contraction. Le phénol utilisé comme témoin d'inhibition de la contraction donne 0% de contraction. L'analyse quantitative par observation en microscopie électronique des gels contractés après 72 heures en présence et en l'absence de l'extrait I 1844 montre des cellules actives qui semblent en phase de synthèse protéique. Le traitement par l'extrait I 1844 ne semble pas modifier sensiblement le nombre et la taille des fibres.

L'extrait I 1844 est efficace dès la dose de 0,1 %. Il augmente l'activité des fibroblastes qui se traduit par une augmentation de la contraction du gel de collagène et du nombre de fibres à la plus forte concentration seulement.

La caractérisation de l'effet de l'extrait I 1844 sur l'activation de la collagénase a été réalisé comme suit.

Le but de cette étude est de tester l'effet de l'extrait I 1844 sur l'activation de la collagénase. On utilise pour cela un gel de collagène réalisé par le mélange de fibroblastes et de collagène tritié. Dans cet environnement, les fibroblastes réorganisent, remodèlent et contractent le collagène pour produire une structure de type dermique. Pour cela, les fibrobtastes produisent de la collagénase qui est relarguée dans le milieu de culture sous la forme latente qui peut être activée sous l'action de protéinases. L'activité de la collagénase est mesurée par sa capacité à digérer un substrat collagénique tritié en peptides radiocatifs solubles. La collagénase latente est activée par la trypsine traitée à la 1,1-tosylamide-2-phényléthyl-chlorométhyl cétone (TPCK). La lignée cellulaire utilisée est la souche MRC5, fibroblastes issus de poumon embryonnaire humain. Ils sont cultivés en milieu BME (Basal Eagle Medium) avec Glutamax I contenant 10% de SVF, 1% de penicilline-streptomycine, 1% de fungizone et 1% de NEAA (acides aminés non essentiels) Gibco®.

Le collagène utilisé est du collagène de type I issu de tendons de queue de rat, en solution à 2 mg/ml dans l'acide acétique. Le collagène marqué au tritium est en solution à 0,3 mg/ml dans l'acide acétique et son activité est de 0,06 mCi/ml (Dupont Net-660).

**Méthode d'obtention des gels**

***Préparation de la suspension cellulaire***

Après trypsinisation d'une culture de MRC5 obtenue sur une boîte de Pétri de 75 cm$^2$ de MRC5, la suspension cellulaire est préparée dans du DMEM contenant 10% de SVF et les antibiotiques à raison de $1.10^6$ cellules/ml.

La préparation du mélange se fait *sur glace* afin de retarder la gélification.

Les différents constituants sont ajoutés sous agitation dans l'ordre et les proportions suivants :

| | |
|---|---|
| DMEM 2 X | 400 µl |
| Collagène type I tritié | 300 µl |
| NaOH 0,1 N | 100 µl |
| SVF | 100 µl |
| Suspension cellulaire | 100 µl |

200 µl de ce mélange bien homogénéisé sont répartis dans une plaque de 24 puits et incubés à 37°C. La radioactivité est de 0,4 µCi/puits.

Une heure après, 500 µl de milieu DMEM + 3 % SVF sont déposés dans chaque puits.

Toutes les 24 heures pendant 7 jours, l'activité de la collagénase activée et latente est mesurée. Pour cela, 500 µl de milieu sont prélevés dans chaque puits et mis à compter en scintillation liquide. 300 µl de solution de trypsine-TPCK à 25 mg/ml dans du DMEM sont déposés dans chaque puits et incubés à 37°C pendant 15 minutes; L'action de la trypsine est arrêtée par addition de 200 µl de solution d'inhibiteur de trypsine à 100 mg/ml dans de l'eau distillée. 500 µl de milieu sont prélevés et mis à compter en scintillation liquide. 500 µl de milieu DMEM + 3 % SVF sont rajoutés dans chaque puits et la plaque est remise à incuber pour 24 heures supplémentaires.

L'extrait I 1844 est dilué dans le milieu DMEM 2X lors de l'obtention du gel de façon à obtenir les concentrations finales de 0,1 % et 0,5 %.

L'extrait I 1844 ne modifie pas l'activité de la collagénase totale. Les proportions respectives de collagénase latente et activée ne sont pas modifiées par rapport au contrôle à un temps donné, bien qu'une augmentation de la collagénase latente soit observée au cours du temps aussi bien au niveau des contrôles qu'au niveau des traités. Le produit n'entraîne pas d'activation de la collagénase.

Dans la préparation des compositions cosmétiques selon la présente invention, les extraits I 1844 ainsi constitués sont mélangés aux solvants aqueux ou non et aux diluants conventionnels compatibles avec une utilisation topique ainsi qu'avec les composants actifs de la composition même. Solvants et /ou diluants appropriés seront choisis selon leur capacité à véhiculer le composant actif de l'extrait de l'invention dans les structures cutanées épidermiques et dermiques.

Les extraits I 1844 de l'invention se sont, en plus, montrés complètement dépourvus de génotoxicité dans les tests de Ames et de la réparation de l'ADN.

Leur stabilité est compatible avec leur utilisation dans des compositions cosmétiques.

Les compositions cosmétiques de la présente invention contiennent l'extrait I 1844 en pourcentages compris de 0,00001 % à 5 % en poids par rapport au poids total de la composition, en mélange avec les excipients communément utilisés pour la préparation de formulations cosmétiques à appliquer sur la peau.

Lesdits pourcentages peuvent varier dans l'intervalle indiqué ci-dessus en fonction de l'activité intrinsèque de l'extrait I 1844 inclus dans la composition.

De préférence ledit extrait I 1844 est présent en pourcentages de 0,0001 % à 2 % % en poids par rapport au poids total de la composition.

Dans la préparation des compositions selon la présente invention, l'extrait I 1844 est mélangé aux solvants aqueux ou non et aux diluants conventionnels compatibles avec une utilisation sur la peau ainsi qu'avec d'autres composants de la composition même. Solvants et /ou diluants appropriés sont choisis selon leur capacité à déposer l'extrait I 1844 actif de l'invention sur la peau.

Ces compositions contiennent généralement des excipients ou des additifs choisis parmi les ingrédients habituellement utilisés dans des compositions destinées à une application locale selon la nécessité des formulations particulières envisagées.

Elles peuvent contenir par exemple, des agents épaississants, des adoucissant, des émollients, des stabilisants, des conservateurs, des agents anti-mousse, des tensioactifs, des antioxydants, des colorants et/ou des pigments et/ou d'autres types de charges, des parfums, des silicones, des corps gras divers.

L'extrait I 1844 est toujours de 0,00001 % à 5 % en poids, de préférence de 0,0001 à 2 % en poids par rapport au poids total de la composition, ladite quantité étant calculée par rapport au poids de l'extrait sec.

Les compositions selon la présente invention comprennent, de préférence, un extrait du moût de fermentation de la nouvelle souche I 1844 dans des proportions, en pourcentage poids/poids, qui dépendent du degré d'activité de l'extrait utilisé et donc de la concentration en substance sèche et de l'activité spécifique de cette substance.

Pour obtenir les compositions cosmétiques selon l'invention, on peut convenablement utiliser les extraits bruts obtenus directement de la fermentation sans aucune étape de purification, dans des proportions comprises de 0,00001 à 5%, avantageusement de 0,0001 à 2 % mieux de 0,001 à 2 % en poids par rapport au poids total de la composition, et de préférence de 0,01 à 2% en poids par rapport au poids total de la composition.

Ces pourcentages en poids sont naturellement calculés sur la base du poids d'extrait sec préparé.

Plus particulièrement, les compositions de la présente invention contiennent des extraits plus ou moins purifiés, mis en solution et susceptibles d'être obtenus par fermentation de la souche *Rhodotorula* déposée auprès de la C.N.C.M. de l'Institut Pasteur sous le numéro I 1844, ou de ses mutants producteurs avec des excipients normalement utilisés pour des formulation de ce genre, tels que ceux mentionnés ci-dessus.

La forme des compositions selon la présente invention peut être une émulsion dans laquelle le constituant ou le mélange de constituants, avec un stabilisant éventuel, est associé aux excipients couramment utilisés dans les compositions cosmétiques et compatibles avec lesdits constituants tels que la lanoline ou les huiles végétales, minérales ou synthétiques.

Les compositions de l'invention peuvent être également présentées sous forme de gel dans des excipients appropriés tels que les esters de cellulose ou d'autres agents gélifiants comme les dérivés acryliques et contenir le principe actif sous forme dissoute ou suspendue en microgranules.

Les compositions selon l'invention peuvent aussi prendre la forme d'une lotion ou d'une solution dans laquelle le mélange des constituants est dissout ou microdispersé.

La forme des compositions selon l'invention peut donc être une microdispersion dans un liquide contenant de l'eau ainsi qu'un ou plusieurs agents tensioactifs compatibles. Les dispersions présentent les caractères des microémulsions en particulier, la transparence et la faible viscosité et ont pratiquement l'aspect de solutions vraies. Elles peuvent également être préparées extemporanément.

Une forme intéressante des compositions selon l'invention est un fluide appliqué topiquement par l'intermédiaire d'un support adhésif, ci-après désigné " patch ", ce patch permettant une diffusion contrôlée des composants actifs éventuellement activée par des phénomènes physiques tels que microcourants électriques.

Les compositions cosmétiques de la présente invention peuvent également contenir d'autres composant actifs ayant soit un effet du même type que celui des stimulateurs de la synthèse de collagène, des inhibiteurs de la collagénase ou de l'élastase, des vasoprotecteurs des produits qui contribuent à stimuler la prolifération des kératinocytes, à stimuler la synthèse des constituants de la matrice extra-cellulaire (collagène, élastine, glycosaminoglycanes) ou des produits utiles dans ce type de composition topique tels que des accélérateurs du renouvellement cellulaire, des inhibiteurs de la collagénase ou de l'élastase, des vasoprotecteurs, des antiradicalaires, des actifs permettant de rétablir la fonction barrière cutanée (céramides, acides gras essentiels) des hydratants.

Les compositions de la présente invention jouissent d'une bonne stabilité et peuvent être conservées pendant le temps nécessaire pour l'utilisation à des températures comprises entre -10°C et 60°C sans qu'il y ait sédimentation des constituants ou séparation des phases, ni une diminution de l'activité qui puisse en compromettre l'utilisation.

Ces compositions sont très bien tolérées, elles ne présentent aucune phototoxicité et leur application sur la peau, pour des périodes prolongées n'implique aucun effet secondaire.

Les compositions de la présente invention, dans leurs différentes formes de présentation, peuvent être utilisées comme préparations destinées à prévenir et/ou à lutter contre le vieillissement cutané qu'il soit de nature intrinsèque ou extrinsèque, principalement actinique dans ce cas.

Les compositions cosmétiques à visée anti-âge de la présente invention peuvent être mises en contact avec l'épiderme ou le système pileux ou capillaire de façon à en modifier l'aspect et à les protéger.

Il est bien connu que la fermeté est une qualité essentielle pour garder une peau jeune. Elle constitue en effet un véritable marqueur de vitalité cellulaire et sa disparition conditionne l'évolution des autres signes de vieillissement comme la formation des rides, ou encore l'absence d'éclat.

Tant que le derme garde sa pleine densité et une souplesse élastique, l'épiderme reste également lisse, souple et très tonique. Le vieillissement de la peau est un phénomène complexe qui commence en pleine jeunesse. Les radicaux libres, les facteurs génétiques, le soleil en sont quelques causes, qui sont cependant multiples et variables. L'ensemble des structures de la peau est touché à tous les niveaux et avec le temps les cellules deviennent moins nombreuses. Elles produisent un tissu de moins bonne qualité qui perd sa fermeté originelle et sa nature élastique.

Il a été constaté que le métabolisme des cellules de l'épiderme se ralentit et que sa couche basale perd sa forme sinueuse et devient rectiligne. La population totale des kératinocytes régresse et la couche cornée perd ainsi son efficacité. De plus elle contrôle mal l'évaporation de l'eau. Il a également été constaté que l'activité des fibroblastes, qui constituent les cellules clés du derme, diminue au fil des années. Le collagène produit, devient alors fibreux et rigide. La synthèse de l'élastine baisse de façon encore plus précoce. La nature du derme change ainsi de texture et il s'amincit, s'affaisse et devient moins résistant aux tractions des muscles. Ce relâchement crée des cassures qui constituent ainsi les rides.

La formule lissante restructurante des compositions cosmétiques selon l'invention réinstaure à tous les niveaux de la peau les bases naturelles du processus de fermeté.

Elles répondent aux micro-transformations imperceptibles de la peau qui aboutissent à la perte de fermeté, d'élasticité et de jeunesse de la peau

Les compositions cosmétiques selon l'invention sont aussi destinées aux peaux matures pour lesquelles elles constituent un soin anti-vieillissement. L'extrait I 1844 agit alors sur le facteur de reconstruction de la peau rétablissant ainsi son équilibre physiologique à la fois structurel (fermeté et tonicité) et superficiel (confort et éclat) ou en profondeur (fermeté et tonicité) et en surface (éclat et confort). Les compositions cosmétologiques selon l'invention, vitalisantes ou restructurantes peuvent être utilisées respectivement le jour et/ou la nuit.

Les compositions cosmétiques peuvent également contenir d'autres actifs complémentaires. Il peut s'agir par exemple de vitamines ou d'anti-tâches.

Leurs qualités restructurantes incomparables sont également renforcées et amplifiées par le caractère synergique d'une association avec par exemple un extrait de plancton marin. L'extrait issu de plancton marin permet d'assurer une relance optimale de l'hydratation et de la fermeté de la peau.

On peut également utiliser des phytostimulines telles que des extraits de fruits, comme par exemple, des phytostimulines de pomme choisies pour leurs propriétés anti-radicalaires. Riches en flavones, stérols, acides gras essentiels et vitamines dont la vitamine E, ils constituent de nouveaux anti-radicalaires particulièrement actifs. La peau est alors protégée de façon maximum contre les dangers des radicaux libres générés notamment par les ultraviolets et le stress.

Des huiles perfluorées peuvent également être utilisées dans les compositions cosmétiques selon l'invention, de plus, ces huiles perfluorées ont pour propriété de lisser les aspérités et les creux du micro-relief du grain de la peau. Véritable "moulage" de jeunesse, elles repassent les traits du visage et embellissent visiblement la surface de la peau.

Ainsi, les compositions cosmétiques selon l'invention stimulent le renouvellement des cellules de l'épiderme et la production de collagène et d'élastine, assurent une relance optimale du processus naturel d'hydratation et protègent de façon encore plus complète la peau contre les radicaux libres qui constituent un des facteurs essentiels de son vieillissement. Redynamisé, l'épiderme retrouve alors une meilleure hydratation, la peau apparaît comme rajeunie, plus lisse et plus tonique. Les traits du visage en particulier, se redéssinent avec plus de netteté et la peau semble restructurée de l'intérieur, devenant ainsi visiblement plus ferme.

L'extrait I 1844 contenu dans les compositions cosmétiques ci-dessus agit sur la peau comme un actif fédérateur.

Des essais cliniques ont été réalisés sur 120 femmes âgées de 30 à 60 ans. Elles ont testé pendant 4 semaines les qualités des compositions cosmétiques selon l'invention.

Il a été constaté chez 87% une efficacité visible du produit sur la fermeté, le lissage et l'hydratation de leur peau. Pour 82% leurs grandes qualités cosmétiques ont été appréciées, par exemple : facilité d'application, pénétration rapide, absence totale de film gras ainsi que sensation de bien être et de confort.

D'autres études cliniques ont consisté à mettre en évidence l'efficacité des compositions cosmétiques selon l'invention sur le relief cutané, le réseau microdépressionnaire, les propriétés biomécaniques de la peau et sa luminosité.

La ligne spécifiquement destinée aux peaux matures a permis de constater qu'avec un soin du jour pour peaux sensibles et peaux sèches on obtient une atténuation statistiquement significative des ridules et du microrelief, une amélioration de la netteté et de l'aspect du réseau microdépressionnaire, ainsi qu'une amélioration de la tonicité de la peau.

Le soin du jour pour peaux normales et mixtes a mis en évidence une atténuation statistiquement significative des ridules, une amélioration de la netteté du réseau microdépressionnaire et une amélioration de la tonicité de la peau.

Le soin de nuit anti-rides/raffermissant a montré une atténuation statistiquement significative des rides profondes, des ridules et du microrelief, une amélioration de la netteté et de l'aspect du réseau microdépressionnaire, une amélioration de la fermeté et de la tonicité de la peau.

Le soin intensif régénérant a révélé une atténuation statistiquement significative des ridules et du microrelief et une amélioration de la netteté et de l'aspect du réseau microdépressionnaire.

Ces différents résultats cliniques ont été confirmés par différentes méthodes bien connues de l'homme du métier en cosmétologie comme par exemple, la technique de la microsonde à imagerie résonance magnétique (IRM) permettant la mesure de l'hydratation ; la microscopie à transmission permettant d'évaluer la reconstitution cutanée ; l'échographie à haute résolution permettant de mesurer l'épaisseur cutanée ou encore la spectrométrie de phosphore 31 permettant de mesurer l'activité cellulaire.

L'invention a également pour objet une méthode de traitement cosmétique caractérisée en ce que l'on applique sur l'épiderme et/ou le système pileux ou capillaire une quantité à effet cosmétique de l'extrait I 1844 dans un véhicule à usage cosmétique.

Les EXEMPLES suivants illustrent l'invention sans toutefois la limiter.

**EXEMPLE 1**

Préparation d'un extrait issu de la souche *Rhodotorula* I 1844.

**1.1 Fermentation**

La fermentation de la souche *Rhodotorula* I 1844 a été conduite sur différents milieux de culture.

**1.1.1**

a) On propage la souche en boîtes de Pétri sur un milieu de repiquage.

| | |
|---|---|
| Autolysat de *Saccharomyces Cerevisiae* | 10 g |
| Tryptone | 10 g |
| Glucose | 20 g |
| Agar | 15 g |
| Eau purifiée q.s.p. | 1 l |

On fait incuber la culture pendant 48 heures à 30°C. On obtient ensuite une suspension de levures en ajoutant dans chaque boîte de Pétri 10 ml d'un milieu de reprise approprié ayant la composition suivante :

| | |
|---|---|
| NaCl | 9,00 g |
| KCl | 0,42 g |
| $CaCl_2$ | 0,48 g |
| $NaHCO_3$ | 0,20 g |
| Glycérol | 150,00 g |
| Acide-3[N-morpholino]propanesulphonique (MOPS) | 3,00 g |
| Eau purifiée q.s.p. | 1 l |

b) On utilise 5 ml de cette suspension pour inoculer une fiole Erlenmeyer de 2 litres contenant 500 ml de milieu de culture ayant la composition suivante :

| | |
|---|---|
| Autolysat de *Saccharomyces Cerevisiae* | 10 g |
| Lait peptonisé | 20 g |
| Corn steep | 10 g |
| Glucose | 30 g |
| Solution d'oligoéléments | 10 ml |
| Eau purifiée q.s.p. | 1 l |

où la solution d'oligoéléments est constituée par les composés suivants :

| | |
|---|---|
| $FeSO_4, 7H_2O$ | 1 g |
| $MnSO_4, 4 H_2O$ | 1 g |
| $CuCl_2, 2H_2O$ | 0,025 g |
| $CaCl_2, 2 H_2O$ | 0,1 g |
| $H_3BO_3$ | 0,56 g |
| $(NH_4)_6Mo_{24}, 4 H_2O$ | 0,02 g |
| $ZnSO_4, 7 H_2O$ | 0,2 g |
| Eau purifiée q.s.p. | 1 l |

La culture est développée pendant 48 heures à 30°C dans des fioles Erlenmeyer de 2 litres en agitant à 150 rpm.

**1.1.2.**

a) On utilise 0,2 ml de suspension de levures du lot de semence secondaire pour inoculer une fiole de 500 ml contenant 100 ml de milieu de culture ayant la composition suivante :

| | |
|---|---|
| Autolysat de *Saccharomyces Cerevisiae* | 20 g |
| Tryptone | 20 g |
| Glucose | 20 g |
| Solution d'oligoéléments | 10 ml |

(suite)

| Eau purifiée q.s.p. | 1 l |
|---|---|

La solution d'oligoéléments est identique à celle décrite dans l'EXEMPLE 1.1.1.
La culture est développée pendant 24 heures à 30°C avec une agitation de 200 rpm.

b) On utilise 100 ml de la culture obtenue ci-dessus pour inoculer un fermenteur de 20 litres contenant 12 litres de milieu de composition identique à celui de l'EXEMPLE 1.1.1.b auquel on ajoute 1 ml/l de Struktol® .

La culture est développée à 30°C avec une aération de 1 wm et en faisant varier l'agitation de façon à maintenir une pression en oxygène dissous de 30 %. Le pH est régulé à 5,0 par de l'ammoniac.
A 25 heures d'âge une solution concentrée de glucose est ajoutée afin de prolonger la croissance.
La culture est arrêtée au palier de croissance, à 32 heures d'âge.

**1.1.3.+**

On prépare 100 ml de culture en fiole agitée de la même façon que dans l'EXEMPLE 1.1.2. et on utilise cette culture pour inoculer un fermenteur de 20 litres contenant 12 litres de milieu de culture ayant la composition suivante :

| Autolysat de *Saccharomyces Cerevisiae* | 20 g |
|---|---|
| Tryptone | 20 g |
| Glucose | 30 g |
| Solution d'oligoéléments | 10 ml |
| Struktol® | 1 ml |
| Eau purifiée q.s.p. | 1 l |

La solution d'oligoéléments est identique à celle décrite dans l'EXEMPLE 1.1.1.
La culture est développée dans les mêmes conditions que dans l'EXEMPLE 1.1.2.b.
A 31 heures d'âge une solution de glucose concentrée est ajoutée afin de prolonger la phase de croissance. La culture est arrêtée au palier de croissance à 34 heures d'âge.

**1.1.4**

On prépare quatre cultures en fioles agitées de 2 litres contenant 500 ml de milieu de même composition que celui de l'EXEMPLE 1.1.2.a.
Les quatre fioles sont chacune inoculées par 0,75 ml par fiole de suspension de levure du lot de semence secondaire. Les cultures sont développées pendant 24 heures à 30°C et 200 rpm. Elles sont ensuite réunies pour inoculer un fermenteur de 450 litres contenant 230 litres de milieu ayant la composition suivante :

| Autolysat de *Saccharomyces Cerevisiae* | 30 g |
|---|---|
| Tryptone | 20 g |
| Roférose | 30 g |
| Oligoéléments | 10 ml |
| Struktol | 1 ml |
| Eau purifiée q.s.p. | 1 l |

La solution d'oligoéléments est identique à celle décrite dans l'EXEMPLE 1.1.1.
La culture est développée à 30°C avec une aération de 1 vvm et en faisant varier l'agitation de façon à maintenir une pression en oxygène dissous proche de 30 %.
Le pH est régulé à 5,5 par de l'ammoniac et à 30 heures d'âge une solution de glucose concentrée est ajoutée afin de prolonger la phase de croissance.

La culture est arrêtée au palier de croissance à 37 heures 30 d'âge.

**1.1.5**

On utilise 0,75 ml de suspension de levures du lot de semence secondaire pour inoculer chacune des 6 fioles de 2 litres contenant 500 ml de milieu de culture ayant la composition suivante :

| Autolysat de *Saccharomyces Cerevisiae* | 20 g |
|---|---|
| Hydrolysat de caséïne | 20 g |
| Glucose | 20 g |
| Oligoéléments | 10 ml |
| Eau purifiée q.s.p. | 1 l |

La solution d'oligoéléments est identique à celle décrite dans l'EXEMPLE 1.1.1.
La culture est développée pendant 24 heures à 30°C avec une agitation de 220 rpm.
Les six fioles sont ensuite réunies pour inoculer un fermenteur de 450 litres contenant 300 litres de milieu ayant la composition suivante :

| Autolysat de *Saccharomyces Cerevisiae* | 30 g |
|---|---|
| Hydrolysat de caséïne | 20 g |
| Glucose | 20 g |
| Oligoéléments | 10 ml |
| Struktol | 1 ml |
| Eau purifiée q.s.p. | 1 l |

La solution d'oligoéléments est identique à celle décrite dans l'EXEMPLE 1.1.1.
La culture est développée à 30°C avec une aération de 1 vvm et en faisant varier l'agitation de façon à maintenir une pression en oxygène dissous proche de 30 %.
Le pH est régulé à 5,5 par de l'ammoniac et à 35 heures d'âge une solution de glucose concentrée est ajoutée afin de prolonger la phase de croissance.
La culture est arrêtée au palier de croissance à 40 heures d'âge.

**1.2 Extraction**

**1.2.1**

On réalise l'extraction sur 9 litres de moût obtenu selon l'EXEMPLE 1.1.1. (18 fioles de 2 litres contenant 500 ml de moût).
On centrifuge à 9000 tours/mn (14 000 g) pendant 10 mn et on obtient 154 g de biomasse humide.
Cette biomasse est mise en contact à température ambiante sous agitation pendant une nuit avec 6 litres de mélange dichlorométhane/méthanol (v/v).
La biomasse est ensuite éliminée par filtration et la phase organique est évaporée à sec sous vide à 50°C.
Le résidu d'évaporation (9,8 g) constitue l'extrait sec et il est redissous dans 112 ml de méthanol.
La fraction ainsi obtenue est filtrée sur 0,2 μm et évaluée sur le test biologique de stimulation des kératinocytes, elle présente un indice de stimulation (IS) des kératinocytes de 1,9 quand elle est diluée au 200ème (1/200).

**1.2.2**

On réalise l'extraction sur 12 litres de moût de culture de l'EXEMPLE 1.1.2.
On suit le même protocole que dans l'EXEMPLE 1.2.1. mais on extrait les 1 900 g de biomasse humide obtenue par 80 litres de mélange dichlorométhane/méthanol (50/50). soit en utilisant une proportion mélange solvant / biomasse humide proche de 40 litres mélange solvant pour 1 kg de biomasse humide.
Après une nuit de mise en contact, la biomasse est éliminée par filtration et la phase organique est évaporée a sec sous vide à 50°C.

On obtient 93,8 g d'extrait sec qui sont repris avec 375 g du mélange éthanol/eau, 50/50 (v/v).

La solution à 0,2 g/g d'extrait sec, filtrée sur 0,2 μm, ainsi obtenue présente un indice de stimulation (IS) de 2,1 pour une dilution avec 1/200.

**1.2.3**

On réalise l'extraction sur 11 litres de culture de l'EXEMPLE 1.1.3. On suit le même protocole que dans l'EXEMPLE 1.2.2. et on extrait les 2,2 kg de biomasse humide obtenue avec 80 litres du mélange dichlorométhane/méthanol, 50/50 (v/v).

La biomasse est ensuite éliminée par filtration et la phase organique est évaporée à sec sous vide à 50°C.

On obtient 98 g d'extrait sec que l'on reprend avec 392 g de mélange éthanol/eau (50/50), (v/v) de façon à obtenir une solution à 0,2 g/g.

Après filtration sur 0,2 μm, cette solution est évaluée sur le test biologique de stimulation des kératinocytes, elle présente un indice de stimulation (IS) de 2,8 pour une dilution de 1/200.

**1.2.4**

On réalise l'extraction sur 12 litres de culture préparée de la même façon que celui de l'EXEMPLE 1.1.3, mais dans ce cas pour séparer la biomasse on utilise une centrifugeuse continue. On obtient 1,6 kg de biomasse humide, cette biomasse est traitée par 80 litres de mélange dichlorométhane/méthanol, 50/50 (v/v).

Après une nuit de mise en contact, la biomasse est éliminée par filtration et la phase organique est évaporée à sec sous vide à 50°C.

On obtient 83 g d'extrait sec que l'on reprend avec 747 g de mélange éthanol/eau, 50/50 (v/v) de façon à obtenir une solution à 0,1 g/g en extrait sec.

La solution obtenue est filtrée sur 0,2 μm et présente un indice de stimulation (IS) de 3,5 pour une dilution au 1/100.

**1.2.5**

On réalise l'extraction de 230 litres de culture de l'EXEMPLE 1.1.3. ; après séparation de la biomasse par centrifugeuse continue, on obtient 29 kg de biomasse humide. On traite 8 kg de cette biomasse par 80 litres de mélange dichlorométhane/méthanol. 50/50 (v/v), soit en utilisant une proportion mélange solvant/biomasse humide proche de 10 litres de mélange solvant pour 1 kg de biomasse humide.

Après une nuit de mise en contact, la biomasse est éliminée par filtration et la phase organique est évaporée à sec sous vide à 50°C.

On obtient après évaporation de la phase organique 314 g d'extrait sec que l'on reprend avec 2 826 g de mélange éthanol/eau, 50/50 (v/v).

La solution ainsi obtenue (0,1 g/g d'extrait sec) présente un indice de stimulation (IS) de 2,8 pour une dilution au 1/100.

**1.2.6**

On réalise l'extraction de 330 litres de culture de l'EXEMPLE 1.1.5. A l'aide d'une centrifugeuse continue, on obtient 54,4 kg de biomasse humide. On traite 27,7 kg de cette biomasse avec 272 litres de mélange dichlorométhane/méthanol, 50/50 (v/v), soit en utilisant une proportion mélange solvant/biomasse humide proche de 5 litres de mélange solvant pour 1 kg de biomasse humide.

Après une nuit de mise en contact, la biomasse est éliminée par filtration et la phase organique est évaporée à sec sous vide à 50°C.

On obtient après évaporation de la phase organique 1447 g d'extrait sec que l'on reprend avec 13023 g de mélange éthanol/eau, 50/50 (v/v) de façon à obtenir une solution à 0,1 g en extrait sec. Après filtration sur 0,2 g cette solution est évaluée sur le test biologique de stimulation des kératinocytes, elle présente un indice de stimulation de 2,6 pour une dilution au 1/100 ème.

Dans les exemples suivants, les quantités sont indiquées en pourcentages en poids.

| EXEMPLE 2 : CREME DE JOUR POUR LE VISAGE | |
|---|---|
| CARBOMER | 0.15 |

(suite)

| EXEMPLE 2 : CREME DE JOUR POUR LE VISAGE | |
|---|---|
| TRIETHANOLAMINE | 0.1 |
| PROPYLENE GLYCOL | 5 |
| GOMME XANTHANE | 0.2 |
| GLYCERINE | 3 |
| STEARATE DE GLYCEROL | 3 |
| STEARATE DE PEG | 2 |
| STEARETH 20 | 2 |
| PALMITATE D'OCTYLE | 10 |
| ALCOOL CETYLIQUE | 2 |
| ALCOOL STEARYLIQUE | 0.25 |
| HUILE PERELUOREE | 0.2 |
| CHOLESTEROL | 0.5 |
| CIRE D'ABEILLE SYNTHETIQUE | 5 |
| DIMETHICONE | 5 |
| EXTRAIT PLANCTON MARIN | 0.5 |
| EXTRAIT DE POMME | 3 |
| BISABOLOL | 0.5 |
| ACETATE DE TOCOPHEROL | 1 |
| EXTRAIT PEPINS DE RAISIN | 0.5 |
| CONSERVATEUR | 0.8 |
| PARFUM | 0.5 |
| EXTRAIT I 1844 | 0.1 |
| EAU DEMINERALISEE q.s.p. | 100 |

| EXEMPLE 3 : CONCENTRE DE SOIN POUR LE VISAGE | |
|---|---|
| BUTYLENE GLYCOL | 5 |
| CARBOMER | 0.2 |
| TRIETHANOLAMINE | 1 |
| POLYSORBATE 60 | 0.8 |
| SORBITAN STEARATE | 0.6 |
| HUILE DE VASELINE | 2 |
| LANOLINE | 0.3 |
| DIPELARGONATE DE | 5 |
| TRIGLYCERIDE | 5 |
| ALCOOL CETYLIQUE | 0.25 |
| DICAPRYLATE/ DICAPRATE DE | 5 |
| STEARATE DE GLYCEROL | 3.00 |

# EP 1 054 660 B1

(suite)

**EXEMPLE 3 : CONCENTRE DE SOIN POUR LE VISAGE**

| | |
|---|---|
| STEROL VEGETAL | 0.5 |
| DIMETHICONE | 0.5 |
| ACIDE STEARIQUE | 2 |
| PANTHENOL | 0.5 |
| HYALURONATE DE SODIUM | 0.05 |
| BISABOLOL | 0.1 |
| ACETATE DE TOCOPHEROL | 1 |
| EXTRAIT GERME DE SOJA | 0.5 |
| CONSERVATEUR | 0.8 |
| PARFUM | 0.5 |
| EXTRAIT I 1844 | 0.25 |
| EAU DEMINERALISEE q.s.p. | 100 |

**EXEMPLE 4 : EMULSION FLUIDE POUR LE VISAGE**

| | |
|---|---|
| BUTYLENE GLYCOL | 5 |
| GOMME XANTHANE | 0.2 |
| STEARATE DE GLYCEROL | 1 |
| STEARATE DE PEG | 1 |
| TRIGLYCERIDE | 5 |
| ALCOOL CETYLIQUE | 0.5 |
| OXYDE DE TITANE | 1 |
| DIMETHICONE | 3 |
| POLYACRYLAMIDE | 5 |
| CERAMIDES | 1 |
| PANTHENOL | 1 |
| HYALURONATE DE SODIUM | 0.05 |
| BISABOLOL | 0.1 |
| ACETATE DE TOCOPHEROL | 1 |
| CONSERVATEUR | 0.7 |
| PARFUM | 0.5 |
| EXTRAIT I 1844 | 0.15 |
| EAU DEMINERALISEE q.s.p. | 100 |

**EXEMPLE 5 : LOTION**

| | |
|---|---|
| PEG 32 | 5 |
| TAMPON PHOSPHATE | 0.1 |
| DIMETHICONE COPOLYOL | 5 |

(suite)

| EXEMPLE 5 : LOTION | |
|---|---|
| POLYSORBATE 20 | 0.1 |
| PROPYLENE GLYCOL | 10 |
| EXTRAIT PLANCTON MARIN | 1 |
| EXTRAIT DE POMME | 5 |
| BISABOLOL | 0.5 |
| ACETATE DE TOCOPHEROL | 0.5 |
| EXTRAIT PROTEINE DE BLE | 2 |
| CONSERVATEUR | 1 |
| PARFUM | 0.3 |
| FXTRAIT I 1844 | 0.1 |
| EAU DEMINERALISEE q.s.p. | 100 |

| EXEMPLE 6 : GEL | |
|---|---|
| CARBOMER | 1 |
| NEUTRALISANT | 0.25 |
| PROPYLENE GLYCOL | 5 |
| PANTHENOL | 1 |
| EXTRAIT D'ALOE VERA | 1 |
| EXTRAIT DE BLEUET | 5 |
| EXTRAIT DE LEVURE | 0.5 |
| EXTRAIT DE BLE | 0.25 |
| HYALURONATE DE SODIUM | 0.01 |
| CONSERVATEUR | 0.7 |
| EXTRAIT I 1844 | 0.05 |
| EAU DEMINERALISEE q.s.p. | 100 |

| EXEMPLE 7 : MASQUE CREME | |
|---|---|
| CARBOMER | 0.5 |
| TRIETHANOLAMINE | 0.5 |
| STEARATE DE SORBITAN | 1.5 |
| STEARATE DE PEG | 3 |
| ALCOOL CETYLIQUE | 5 |
| HUILE VEGETALE | 20 |
| OXYDE DE TITANE | 0.7 |
| GLYCERINE | 7 |
| TOCOPHEROL | 0.75 |
| EXTRAIT DE POMME | 3 |

(suite)

| EXEMPLE 7 : MASQUE CREME | |
|---|---|
| BISABOLOL | 0.5 |
| PALMITATE DE RETINOL | 0.5 |
| HYALURONATE DE SODIUM | 0.02 |
| CONSERVATEUR | 1 |
| PARFUM | 0.3 |
| EXTRAIT I 1844 | 0.1 |
| COLORANTS | |
| EAU DEMINERALISEE q.s.p. | 100 |

| EXEMPLE 8 : MASQUE A RINCER | |
|---|---|
| COPOLYMERE ACRYLIQUE | 1 |
| TRIETHANOLAMINE | 1 |
| GLYCERINE | 15 |
| C14-C16 OLEFINS SULFONATES | 5 |
| ETHER DE PEG ET COCOATE DE | 2 |
| EXTRAIT PLANCTON MARIN | 1 |
| EXTRAIT DE POMME | 5 |
| EXTRAIT DE GERME DE SOJA | 0.5 |
| ASCORBYLE PHOSPHATE DE | 3 |
| EXTRAIT PROTEINE DE SOIF | 2 |
| CONSERVATEUR | 0.8 |
| PARfUM | 0.5 |
| EXTRAIT I 1844 | 0.15 |
| EAU DEMINERALISEE q.s.p. | 100 |

## Revendications

1. Composition cosmétique comprenant en mélange avec un excipient pour préparations cosmétiques un extrait à activité stimulatrice de la production d'IL-6 par les kératinocytes qui est un produit provenant de la fermentation d'un micro-organisme.

2. Composition selon la revendication 1, **caractérisée en ce que** l'extrait à activité stimulatrice de la production d'IL-6 par les kératinocytes est un produit provenant de la fermentation d'une levure.

3. Composition selon la revendication 2 **caractérisée en ce que** l'extrait à activité stimulatrice de la production d'IL-6 par les kératinocytes est obtenu par fermentation d'une souche de *Rhodotorula.*

4. Composition selon la revendication 3 **caractérisée en ce que** l'extrait à activité stimulatrice de la production d'IL-6 par les kératinocytes est susceptible d'être obtenu par fermentation de la souche, *Rhodotorula* SEBR 2002 déposée auprès de la C.N.C.M. de l'Institut Pasteur sous le numéro I 1844, ou de ses mutants producteurs.

5. Souche *Rhodotorula* SEBR 2002, déposée auprès de la C.N.C.M. de l'Institut Pasteur sous le numéro I 1844 et ses mutants producteurs.

**6.** Procédé de préparation d'un produit extrait à activité stimulatrice de la production d'IL-6 par les kératinocytes, **caractérisé en ce que** l'on cultive une souche selon la revendication 5, sur un milieu de fermentation et dans des conditions classiques jusqu'à l'obtention, dans le moût de fermentation, d'une activité stimulatrice de la production d'IL-6 par les kératinocytes.

**7.** Composition à activité stimulatrice de la production d'IL-6 par les kératinocytes obtenue selon le procédé de la revendication 6.

**8.** Composition selon la revendication 7 obtenue selon le procédé de la revendication 6, dans lequel on utilise la biomasse telle quelle ou concentrée.

**9.** Composition selon la revendication 7 **caractérisée en ce qu'**elle présente une activité stimulatrice de la production d'IL-6 par les kératinocytes avec un indice de stimulation IS compris entre 1,2 et 6.

**10.** Composition selon l'une des revendications 7 à 9, **caractérisée en ce qu'**elle est obtenue par extraction de la biomasse, évaporation des solvants et de l'eau et dilution de l'extrait sec dans un solvant compatible avec une utilisation cosmétique.

**11.** Utilisation d'un composé à activité stimulatrice de la production d'IL-6 par les kératinocytes provenant de la fermentation d'un microorganisme pour la fabrication d'une composition cosmétique destinée à lutter contre le vieillissement cutané.

**12.** Utilisation selon la revendication 11, **caractérisée en ce que** le composé est obtenu par fermentation d'une levure.

**13.** Utilisation selon la revendication 12, **caractérisée en ce que** le composé est obtenu par une souche de *Rhodotorula*, notamment *Rhodotorula* SEBR 2002, déposée auprès de la C.N.C.M. de l'Institut Pasteur sous le numéro I 1844, ou de ses mutants producteurs.

**14.** Utilisation d'une souche de *Rhodotorula,* notamment *Rhodotorula* SEBR 2002, déposée auprès de la C.N.C.M. de l'Institut Pasteur sous le numéro I 1844, ou de ses mutants producteurs pour la préparation d'un composé à activité stimulatrice de la production d'IL-6 par les kératinocytes.

**Patentansprüche**

**1.** Kosmetische Zubereitung umfassend in Mischung mit einem Trägermaterial für kosmetische Präparate einen Extrakt mit stimulierender Wirkung auf die Produktion von IL-6 durch Keratinozyten, bei dem es sich um ein Produkt handelt, welches durch Fermentierung eines Mikroorganismus erzeugt worden ist.

**2.** Zubereitung nach Anspruch 1, **dadurch gekennzeichnet, daß** der Extrakt mit stimulierender Wirkung auf die Produktion von IL-6 durch Keratinozyten ein durch die Fermentierung einer Hefe erzeugtes Produkt ist.

**3.** Zubereitung nach Anspruch 2, **dadurch gekennzeichnet, daß** der Extrakt mit stimulierender Wirkung auf die Produktion von IL-6 durch Keratinozyten durch Fermentierung eines *Rhodotorula*-Stammes erhalten worden ist.

**4.** Zubereitung nach Anspruch 3, **dadurch gekennzeichnet, daß** der Extrakt mit stimulierender Wirkung auf die Produktion von IL-6 durch Keratinozyten erhältlich ist durch Fermentierung des Stammes *Rhodotorula* SEBR 2002, hinterlegt bei dem C.N.C.M. des Institut Pasteur unter der Nummer I 1844, oder eines seiner produzierenden Mutanten.

**5.** Stamm *Rhodotorula* SEBR 2002, hinterlegt bei dem C.N.C.M. des Institut Pasteur unter der Nummer I 1844 und seine produzierenden Mutanten.

**6.** Verfahren zur Herstellung eines Extraktprodukts mit stimulierender Wirkung auf die Produktion von IL-6 durch Keratinozyten, **dadurch gekennzeichnet, daß** man einen Stamm nach Anspruch 5 auf einem klassischen Fermentierungsmedium und unter üblichen Bedingungen züchtet, bis man in der Fermentierungsbrühe eine stimulierende Aktivität bezüglich der Produktion von IL-6 durch Keratinozyten erhält.

**7.** Zubereitung mit stimulierender Wirkung auf die Produktion von IL-6 durch Keratinozyten, erhalten nach dem Verfahren des Anspruchs 6.

**8.** Zubereitung nach Anspruch 7, erhalten nach dem Verfahren des Anspruchs 6, worin man die Biomasse so, wie man sie erhalten hat, oder in konzentrieter Form verwendet.

**9.** Zubereitung nach Anspruch 7, **dadurch gekennzeichnet, daß** sie eine stimulierende Aktivität für die Produktion von IL-6 durch Keratinozyten mit einem Stimulierungsindex IS zwischen 1,2 und 6 aufweist.

**10.** Zubereitung nach einem der Ansprüche 7 bis 9, **dadurch gekennzeichnet, daß** sie erhalten worden ist durch Extraktion der Biomasse, Verdampfen der Lösungsmittel und des Wassers und Verdünnen des Trockenextrakts mit einem Lösungsmittel, welches für eine kosmetische Anwendung verträglich ist.

**11.** Verwendung einer Verbindung mit stimulierender Aktivität auf die Produktion von IL-6 durch Keratinozyten erhalten durch Fermentierung eines Mikroorganismus zur Herstellung einer kosmetischen Zubereitung zur Bekämpfung des Alterns der Haut.

**12.** Verwendung nach Anspruch 11, **dadurch gekennzeichnet, daß** die Verbindung durch Züchten einer Hefe erhalten worden ist.

**13.** Verwendung nach Anspruch 12, **dadurch gekennzeichnet, daß** die Verbindung erhalten worden ist aus einem *Rhodotorula*-Stamm, insbesondere *Rhodotorula* SEBR 2002, der beim C.N.C.M. des Institut Pasteur unter der Nummer I 1844 hinterlegt worden ist, oder seiner produzierenden Mutanten.

**14.** Verwendung eines *Rhodotorula*-Stammes, insbesondere *Rhodotorula* SEBR 2002, hinterlegt beim C.N.C.M. des Institut Pasteur unter der Nummer I 1844, oder seiner produzierenden Mutanten für die Herstellung einer Verbindung mit stimulierender Wirkung auf die Produktion von IL-6 durch Keratinozyten.

**Claims**

**1.** Cosmetic composition comprising, mixed with an excipient for cosmetic preparations, an extract with stimulatory activity on IL-6 production by keratinocytes, which is a product originating from the fermentation of a microorganism.

**2.** Composition according to Claim 1, **characterized in that** the extract with stimulatory activity on IL-6 production by keratinocytes is a product originating from the fermentation of a yeast.

**3.** Composition according to Claim 2, **characterized in that** the extract with stimulatory activity on IL-6 production by keratinocytes is obtained by fermentation of a strain of *Rhodotorula.*

**4.** Composition according to Claim 3, **characterized in that** the extract with stimulatory activity on IL-6 production by keratinocytes can be obtained by fermentation of the strain *Rhodotorula* SEBR 2002 which is deposited with the C.N.C.M. of the Pasteur Institute under the number I 1844, or of its producer mutants.

**5.** Strain *Rhodotorula* SEBR 2002, which is deposited with the C.N.C.M. of the Pasteur Institute under the number I 1844, and its producer mutants.

**6.** Method for preparing an extract product with stimulatory activity on IL-6 production by keratinocytes, **characterized in that** a strain according to Claim 5 is cultured on a fermentation medium and under conventional conditions until a stimulatory activity on IL-6 production by keratinocytes is obtained in the fermentation must.

**7.** Composition with stimulatory activity on IL-6 production by keratinocytes, obtained according to the method of Claim 6.

**8.** Composition according to Claim 7 obtained according to the method of Claim 6, in which the biomass is used in unmodified form or concentrated.

**9.** Composition according to Claim 7, **characterized in that** it exhibits a stimulatory activity on IL-6 production by keratinocytes with a stimulation index SI of between 1.2 and 6.

10. Composition according to one of Claims 7 to 9, **characterized in that** it is obtained by extraction of the biomass, evaporation of the solvents and water, and dilution of the dry extract in a solvent which is compatible with cosmetic use.

11. Use of a compound with stimulatory activity on IL-6 production by keratinocytes, originating from the fermentation of a microorganism, for manufacturing a cosmetic composition intended for combating skin ageing.

12. Use according to Claim 11, **characterized in that** the compound is obtained by fermentation of a yeast.

13. Use according to Claim 12, **characterized in that** the compound is obtained with a strain of *Rhodotorula*, in particular *Rhodotorula* SEBR 2002 which is deposited with the C.N.C.M. of the Pasteur Institute under the number I 1844, or of its producer mutants.

14. Use of a strain of *Rhodotorula,* in particular *Rhodotorula* SEBR 2002 which is deposited with the C.N.C.M. of 'the Pasteur Institute under the number I 1844, or of its producer mutants, for preparing a compound with stimulatory activity on IL-6 production by keratinocytes.